# EUROPEAN PATENT SPECIFICATION

(11) **EP 0 888 751 B1**
(45) Date of publication and mention of the grant of the patent: **10.09.2003**
(21) Application number: 98305216.8
(22) Date of filing: 01.07.1998
(51) Int. Cl.: A61B 17/56, A61F 2/46

(54) **Apparatus for releasably attaching operating means to a surgical element or tool**
Apparat zum lösbaren Anbringen eines Betätigungsmittels an einem chirurgischen Element oder Werkzeug
Appareil pour la fixation amovible de moyens d'opération à un élément ou outil chirurgical

(30) Priority: 02.07.1997 GB 9714004
(43) Date of publication of application: 07.01.1999
(73) Proprietor: HOWMEDICA INTERNATIONAL S. DE R.L., Shannon Co. Clare (IE)
(72) Inventor: Péché, Jean-Patrick, 95034 Cergy-Pontoise (FR); Storer, John Andrew, Bayeux (FR); Bree, Jean-Pierre, 14790 Fontaine Etoupefour (FR)
(74) Representative: Bridge-Butler, Alan James

(56) References cited:
- EP-A- 0 380 309
- WO-A-89/04238
- DE-U- 29 509 010
- US-A- 4 921 493
- US-A- 4 963 155

## Description

This invention relates to apparatus for releasably attaching operating means to a surgical element or tool.

The term operating means is used to include devices such as an impact hammer , anvil or handle, and the term surgical element or tool is used to include any tool or implement which can be attached to operating means, for example a proximal impactor, trial stem or a bone rasp.

Proximal impactors are used to impact, for example, bone chippings, into, for example, the intramedullary canal of a femur into which a prosthesis is to be fitted and can have a stem which resembles the stem of the prosthesis which is to be fitted.

In known constructions the impactor has a connection member in the form of an impaction rod which is integral with it, the free end of the rod either acting as an anvil or being adapted for connection to an impaction hammer or similar device. Trial stems can be inserted using an impaction rod connected to a hammer with the distal end bearing against the trial stem but not connected to it.

DE-U-295 09 010 relates to apparatus for holding a broach which can be used in a bone canal and comprises a connecting member which is attached to the broach and which can also be connected to operating means. The means of attachment appear to comprise a plug which can extend into a bore within the broach and which carries a pair of radially extending resilient biased pins which can compress as the plug is inserted into the bore and subsequently extend radially into engagement with openings in the wall of the broach. No way of releasing the pins is described although presumably they can be released by pressing inwardly on their outer ends. A disadvantage of this construction is that there will be a tendency for heavy wear on the walls of the openings in the broach caused by the pressure of the pins, especially if an implement such as a slap-hammer is employed. The method of connecting the two parts together is essentially axial and, as referred to above, after heavy use wear will occur and the connection will become sloppy.

US-A-4 963 155 shows a construction for a set of surgical products which comprise the stem of a femoral component, an interconnecting neck portion and a load carrying ball. The neck portion is attached to the stem by a plug which carries a projecting key. The plug and key engage in an appropriate opening in the stem. In order to retain rotational stability a projecting spline is provided which engages in a U-shaped opening in the neck. There is however no means of locking the neck to the stem and the invention is directed to constructions where a trial prosthesis is used to evaluate fit before implanting the actual hip stem prosthesis implant.

The present invention is intended to provide apparatus which has a more universal use and can be utilised in such a way that it can not only be used on proximal impactors but also trial stems and bone rasps or other elements.

In the present invention the term "proximal impactor" will be used to not only include proximal impactors per se but also trial stems.

The apparatus according to the present invention is defined in claim 1.

With this arrangement the surgical element or tool can be securely attached to the operating means so that it is correctly placed, for example when an impaction hammer is used, to receive the results of the hammering action but can be easily released.

Resiliently biased means can also be included for holding said projecting abutment in position beneath said securing abutment.

The attachment means for releasably attaching the locking element to the connecting member can comprise a radially extending shaped slot provided on said locking element which engages a retaining member on the connecting member.

Alternatively the radially extending slot can be provided on the connecting member and the retaining member on the locking element.

Thus, the radially extending shaped slot in both constructions can have a circumferentially extending component into which the retaining member can be rotated to attach and lock said locking element to the connecting member.

Resiliently biased means can also be provided for holding the retaining member in the rotated position in the slot.

If a proximal impactor is used it can be in the form of a trial stem and include means to receive a ball end, or can include a ball end.

The invention can be performed in various ways but some embodiments will now be described by way of example and with reference to the accompanying drawings in which :
Figure 1 shows a prior art construction in which an impaction hammer is used to drive a proximal impactor into position;
Figure 2 is a side elevation of an connecting member which forms part of the apparatus of the present invention;
Figure 3 is an isometric view of the member shown in Figure 1;
Figure 4 is an isometric view of a locking element for use with the connecting member shown in Figures 2 and 3;
Figure 5 is a cross sectional end elevation on the line V-V of Figure 4 showing part of the construction;
Figure 6 is a side elevation of a trial stem incorporating part of the invention;
Figure 7 is a cross sectional plan view on the line VII-VII of Figure 6;
Figure 8 is a side elevation of a second embodiment;
Figure 9 is a side elevation of a connecting member for another alternative construction;
Figure 10 is a cross-sectional end view on the line X-X of Figure 9;
Figure 11 is a cross-sectional side elevation of a locking element for use with the connecting member shown in Figures 9 and 10;
Figure 12 is an isometric view of the connecting member shown in Figures 9 and 10 and the locking element shown in Figure 11 ready for assembly;
Figure 13 is a side elevation of the parts shown in Figure 12 assembled together;
Figure 14 is a side elevation in part cross-section showing the locking member and locking element shown in Figures 9 to 11 assembled onto a trial stem or proximal impactor;
Figure 15 is a cross-sectional detail view of part of the assembly shown in Figure 14;
Figure 16 is a part cross-sectional view of the assembly shown in Figure 14 applied to an anvil;
Figure 17 is an isometric view of the construction shown in Figure 16; and,
Figure 18 is an isometric view of a connecting member as shown in Figure 3 fitted with an operating handle.

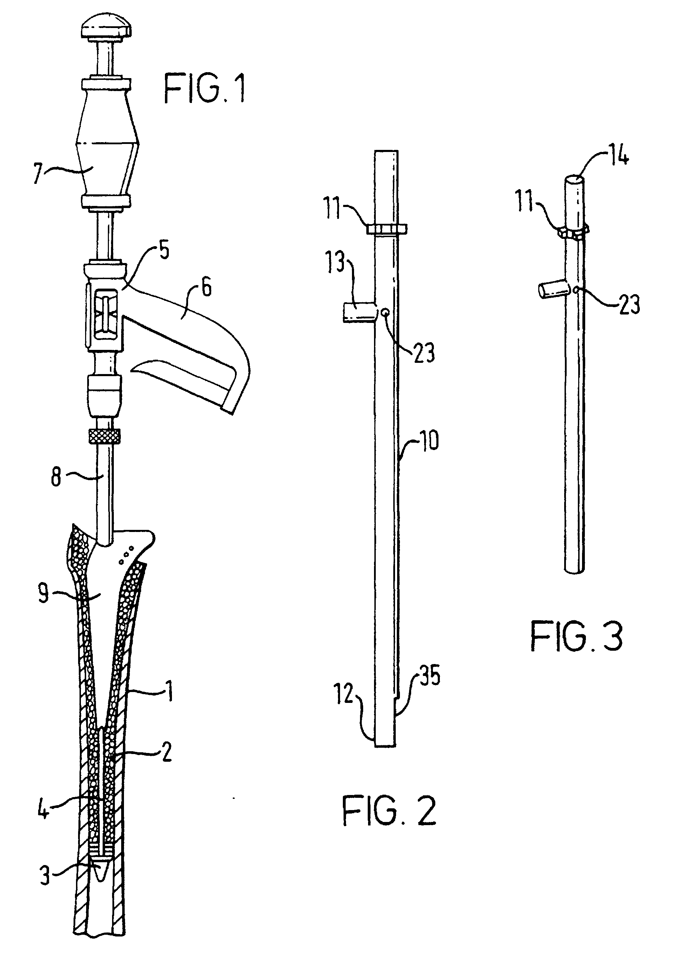

Figure 1 shows a prior art construction for impacting a proximal impactor into bone chippings in the intramedullary canal of a femur.

The femur 1 is filled with impacted bone chippings 2 which are located in place by a bone plug 3. A guide wire 4 is provided onto which the various parts can be threaded in known manner. The impaction hammer 5 has a handle 6 and a slap hammer 7 for providing the impaction effect. The hammer is attached to a connecting member in the form of an impaction rod 8 which is integral with a proximal impactor 9.

A first embodiment of the present invention is shown in Figures 2 to 7 and comprises apparatus for releasably attaching operating means in the form of an impaction hammer, for example of the kind shown in Figure 1, to a surgical element, which in this example is a proximal impactor or a trial stem, to be impacted into bone chippings as described above.

The construction will be described with regard to use with a proximal impactor which, because of the unique construction of the present invention, can also be used as a trial stem.

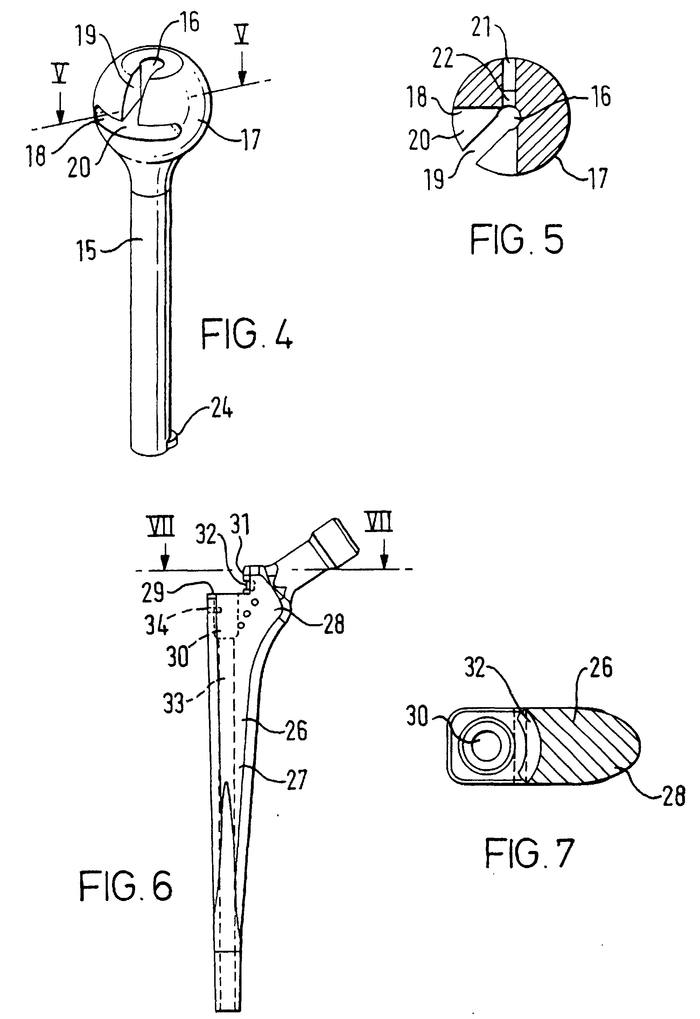

The apparatus comprises a connecting member in the form of an impaction rod 10, as shown in Figure 2 and Figure 3, provided with conventional means for attachment to an impaction hammer of the kind shown in Figure 1. The attachment means comprises a cruciform shaped fitting 11 which is of known type and will not be described in further detail. The distal end 12 of the impaction rod 10 is adapted to engage the proximal end of the impactor shown in Figures 6 and 7 in the manner to be described hereunder. The rod 10 also carries retaining means in the form of a cross member 13 which is shaped to engage a locking element shown in Figure 4.

The impaction rod is also provided with a bore 14 to receive a guide wire of known type.

A locking element for use with the rod 10 comprises a sleeve 15 having a hollow bore 16 which is shaped to slide over the rod 10 and allow it to rotate. The proximal end of the sleeve 15 has an enlarged head 17 in which is located a double keyhole slot 18 which is formed by a radially extending shaped slot 19 which has a circumferentially extending component 20 which extends on either side of the slot 19 and is most clearly shown in Figure 5. The enlarged head 17 is also provided with a radial bore 21 in which is located a spring loaded plunger 22 which can engage depressions 23 on each side of the impaction rod adjacent the cross member 13 and which can co-operate with the plunger 22 when the parts are assembled together to retain the sleeve 15 in predetermined locking positions which are at 90° to each other.

The distal end of the sleeve 15 is provided with a projecting abutment 24.

A proximal impactor in the form of a trial stem 26 which forms part of the invention is shown in Figure 6 and comprises a stem portion 27 and a head portion 28, the proximal end of which is provided with a flat surface 29 and a bore 30 which is of a diameter to accept the distal end 12 of the rod 10 as a push fit. Also located at the proximal end of the stem 26 is a projecting securing abutment 31 which is formed as a slot 32 which is shaped and dimensioned to receive the projecting abutment 24 on the sleeve 15.

The trial stem 26 also has a bore 33 to receive a guide wire in known fashion and a grub screw 34 is provided, the inner end of which projects into the bore 30 where it can co-operate with a flat 35 formed on the distal end of the impaction rod 10.

The parts are assembled together by first sliding the sleeve 15 over the rod 10 from the distal end of the rod until the cross member 13 passes down the slot 19. The assembly is now placed on the trial stem 26 with the distal end 12 of the rod 10 in the bore 30 the grub screw engaging the flat 35 to prevent rotation of the rod 10. The sleeve 15 is now rotated with the cross member 13 passing along the slot 18 and the abutment 24 rotates until it engages in the groove 32 on the trial stem. The resiliently biased pin 22 now acts to hold the retaining member, in the form of the cross member 13, in position in the slot 18 and the impaction rod is securely held onto the trial stem 26. The assembly can now be fitted to the hammer in the usual way.

In order to disassemble the apparatus it is merely necessary to rotate the sleeve 15 against the action of the resiliently biased pin 22 until the cross member 13 is in alignment with the slot 19. In this position the abutment 24 is released from the slot 32 and the parts can be taken apart.

The sleeve and impaction rod can however be held together if desired after removal from the stem 26 by rotating the sleeve further until pin 13 enters the other side of the slot 18 where the second depression 23 engages the retaining pin 22 to hold the parts together.

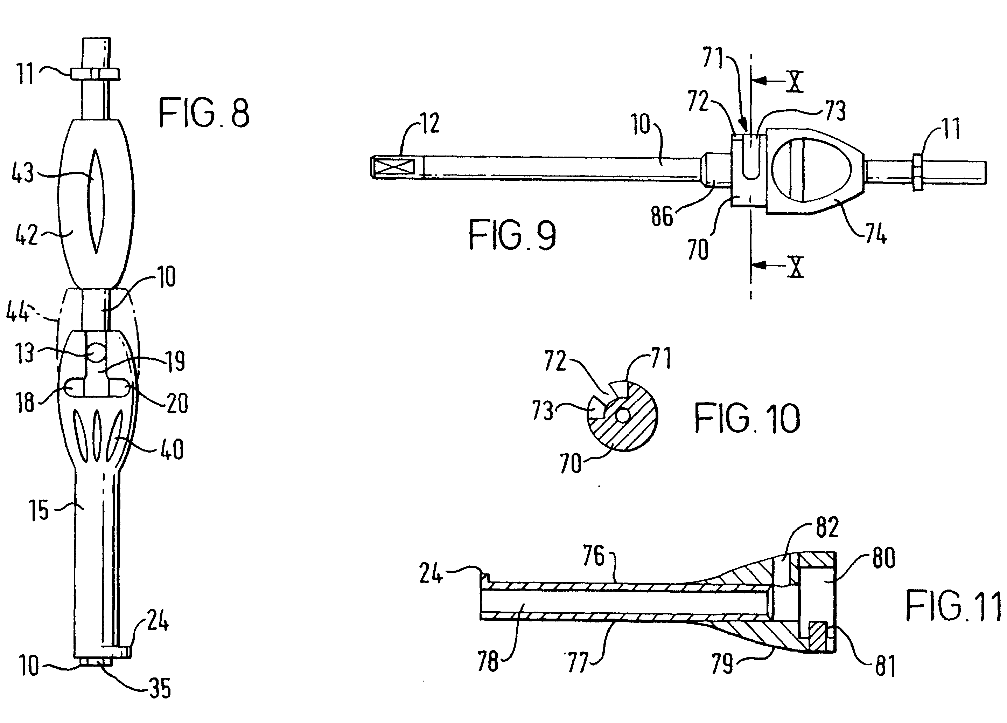

Figure 8 shows a construction which is generally similar to that shown in Figures 2 to 6 and the same reference numerals are used to indicate similar parts. In this construction however the sleeve 5 is of a different shape and carries serrations 40 to assist in its manipulation. A hand grip 42 is also rigidly attached or provided on the impaction rod 10 and this also carries serrations 43.

This embodiment acts in a similar manner to the construction shown in Figures 2 to 6. In Figure 8 the sleeve 15 is shown in position ready for attachment to the trial stem 26. The distal end of the rod 10 is pushed into the bore 30 and at the same time the sleeve 15 rises until the cross member 13 is aligned with the slot 18. The sleeve can now be rotated so that the abutment 24 locks in the groove 32. This raised position of the sleeve is indicated by broken lines 44.

The arrangement is such that the proximal end of the sleeve 15 now bears against the distal end of the hand grip 42 so that when the impaction hammer is operated impaction loads are not only communicated through the impaction rod 10 but also through the hand grip 42 and the sleeve 14 to the trial stem 26.

Figures 9 to 14 show another form of the present invention and the same reference numerals are used to define similar parts to those shown in Figures 1 to 9. In this construction an impaction rod 10 is again provided with conventional means for attachment to an impaction hammer which includes a cruciform shaped fitting 11.

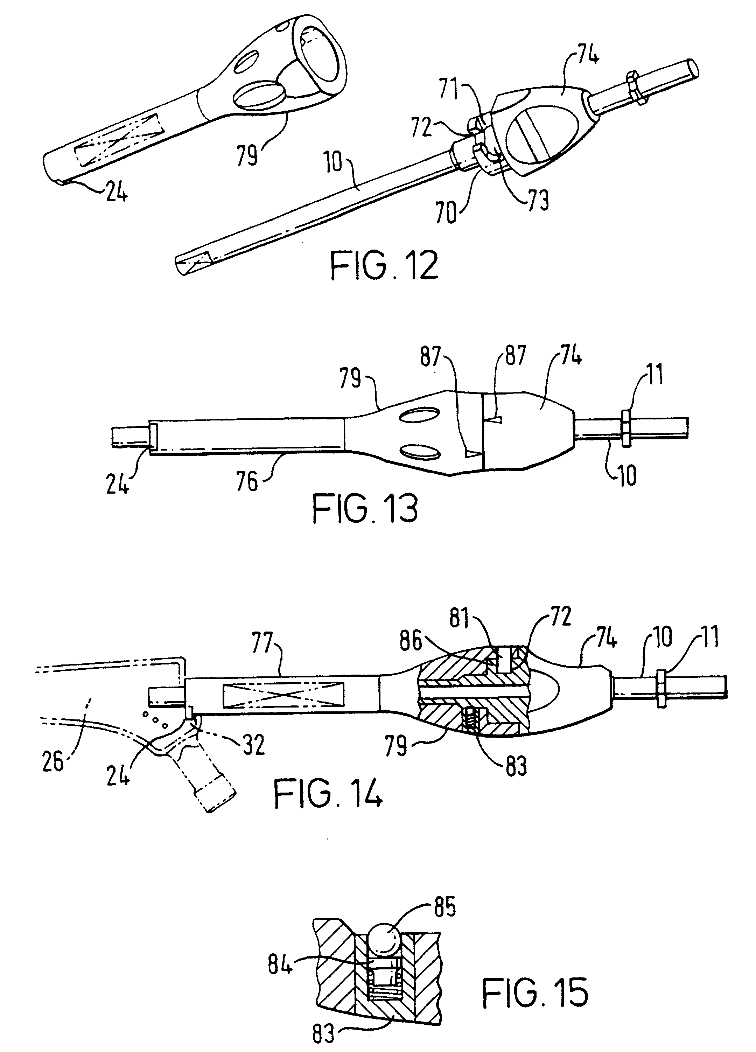

The distal end 12 of the rod is again adapted to engage the proximal end of the impactor and the rod carries retaining means which, in this construction, are provided by an enlarged portion 70 in which is located a double keyhole slot 71 and which comprises a radially extending shaped slot 72 which has a circumferentially extending component 73 which extends on either side of the slot 72 and which is most clearly shown in Figure 12. It will be appreciated that this slot is similar to keyhole slot 18 provided in the locking element shown in Figure 4. The enlarged portion 70 is also provided with a shaped end portion 74.

A locking element 76 is provided for use with the rod 10 and this comprises a sleeve 77 having a hollow bore 78 which is shaped to slide over the rod 10. The proximal end of the locking element has an enlarged portion 79 in which is provided an enlarged socket 80, the dimensions of which are arranged so that it is an easy sliding fit over the enlarged portion 70 of the rod 10. Projecting radially inwardly is an abutment pin 81 which is dimensioned and adapted to pass into the keyhole slot 71 when the parts are assembled. A radially extending bore 82 carries a mounting 83 in which is located a spring loaded plunger 84 and ball 85 which are adapted to engage depressions (not shown) on a boss 86 provided on the rod 10.

The distal end of the locking element is again provided with a projecting abutment 24 similar to that shown in Figure 4.

With this construction the retaining member is provided on the locking element and is in the form of the pin 81.

It will be appreciated that the keyhole slot and retaining member are thus reversed when considered in relation to the construction shown in Figures 1 to 6 but the operating action is similar.

Once again, the parts are assembled together by first sliding the sleeve 77 over the rod 10 from the distal end of the rod until the retaining member 81 passes down the slot 72. The assembly is now placed on the trial stem 26 with the distal end of the rod in the bore 30 in exactly a similar manner to that described with regard to Figures 1 to 6. The sleeve 77 is now rotated so that the pin 81 passes along the slot 72 and the abutment 24 rotates until it engages in the groove 32 on the trial stem. The resiliently biased ball 85 now acts to hold the retaining member in position in the slot 77 and the impaction rod is securely held onto the trial stem 26. The assembly can now again be fitted to the hammer in the usual way.

Disassembly of the apparatus is the reverse to assembly.

Once again the sleeve and impaction rod can be held together if desired after removal from the stem 26 by rotating the sleeve further until the pin 81 enters the other side of the slot and when the second depression (not shown) in the boss 86 engages the retaining ball 85 to hold the parts together.

In order to clearly indicate the positions of the parts markers 87, as shown in Figure 13, are provided. These indicate the relative rotary positions of the rod 10 and the locking element 76, the position in which the pins are in alignment indicated the disengagement position of the two parts.

In all the constructions described above the impaction rod 10 is shown with conventional means for attachment to an impaction hammer but it will be appreciated that the same means can alternatively be used for attachment to an anvil. In certain circumstances, for example with bone rasps, it may be preferable to use such a device which basically comprises an anvil head which can be struck by a mallet, hammer or similar device wielded by the surgeon. When using such an anvil it can be removably attached to the rod 10 in a similar manner to the impaction hammer 5 shown in Figure 1.

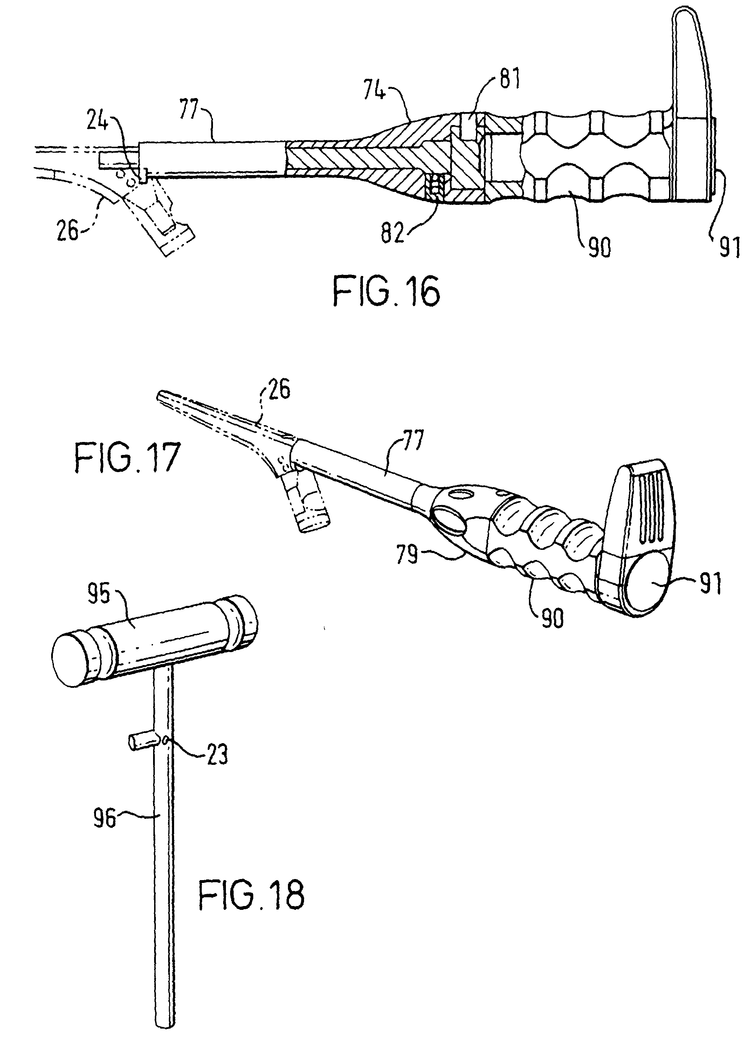

If desired however an impact anvil can be formed as part of the impact rod 10 and Figures 16 and 17 show such a construction. In this arrangement the same reference numerals are used to indicate the same parts as those shown in Figures 10 to 14 but the proximal end and end portion 74 of the impaction rod are replaced by a hand grip 90 embodying an anvil 91. If desired such a hand grip and anvil, or even anvil alone, can be formed on the proximal end of the impaction rod shown in Figures 1 to 8 to replace the proximal end of the rod and the cruciform shaped fitting 11.

Figure 18 shows operating means in the form of a handle 95 which is rigidly secured to a connecting member in the form of a rod 96 which is similar to the impact rod 10 shown in Figure 3. The apparatus works in a similar manner to that described with regard to Figures 1 to 6 but is particularly designed to operate with a bone rasp (not shown) which is connected to the proximal end of the rod 96 in a similar manner to the proximal impactor 26 shown in Figures 6 and 7.

It will be appreciated that the above constructions are merely examples of means for releasably securing the impaction rod to a proximal impactor and other arrangements could be employed.

## Claims

1. Apparatus for releasably attaching operating means (5) to a surgical element or tool (26), the proximal end of which is formed with a bore (30) and a securing abutment (31) said apparatus comprising a connecting member (10) the proximal end of which is attached to or has attachment means (11) for securing it to the operating means (5) and a distal end (12) which is adapted to fit into the bore (30) in the surgical element or tool (26), **characterised by**
including a sleeve shaped locking element (15)(76) which can slide and rotate over the connecting member (10),
the distal end of said locking element (15)(76) having locking means formed by a projecting abutment (24) which is shaped to engage beneath said securing abutment (31) on the element or tool (26) when the locking element (15)(76) is assembled over the connecting member (10), the distal end (12) of the connecting member (10) is located in the bore (30), and the locking element (76)(15) is rotated, and attachment means (19,13)(71,81) for releasably attaching said sleeve shaped locking element (15)(76) to said connecting member (10).

2. Apparatus as claimed in claim 1 **characterised by** including resiliently biased means for holding said projecting abutment (24) in position beneath said securing abutment (31).

3. Apparatus as claimed in any one of preceding claims 1 or 2 **characterised in that** the attachment means (19)(13) for releasably attaching said locking element to said connecting member (10) comprises a radially extending shaped slot (19) which can engage a retaining member (13) on said connecting member (10).

4. Apparatus as claimed in any one of preceding claims 1 or 2 **characterised in that** the attachment means (71)(81) for releasably attaching said locking element (76) to said connecting member (10) comprises a radially extending shaped slot (71) provided on said connecting member (10) and which can engage a retaining member (81) on said locking element (36).

5. Apparatus as claimed in claim 3 or claim 4 **characterised in that** said radially extending shaped slot (19) in said locking element (10) or the slot (71) on said connecting member (10) has a circumferentially extending component (73) into which the retaining member (13) can be rotated to attach and lock said locking element (101)(76) on said connecting member (10).

6. Apparatus as claimed in claim 5 **characterised by** including resiliently biased means (22) for holding said retaining member (13) in the rotated position in said slot (19)(71).

7. Apparatus as claimed in any one of the preceding claims **characterised in that** the operating means comprises a handle (90), anvil (91) or impaction hammer (5).

8. Apparatus as claimed in any one of the preceding claims **characterised in that** the surgical instrument or tool comprises a proximal impactor, a trail stem or a bone rasp or file.

## Patentansprüche

1. Vorrichtung zum lösbaren Befestigen einer Bedienungseinrichtung (5) an einem chirurgischen Element oder Instrument (26), dessen proximales Ende mit einer Bohrung (30) und einem Sicherungsanschlag (31) ausgebildet ist, wobei die Vorrichtung ein Verbindungsteil (10), dessen proximales Ende an der Bedienungseinrichtung (5) befestigt ist oder Befestigungseinrichtungen (11) aufweist, um es an der Bedienungseinrichtung (5) zu befestigen, sowie ein distales Ende (12) umfasst, das angepasst ist, um in die Bohrung (30) im chirurgischen Element oder Instrument (26) zu passen, **dadurch gekennzeichnet, dass**
es ein hülsenförmiges Verriegelungselement (15)(76) einschließt, das sich über dem Verbindungsteil (10) verschieben und drehen kann,
das distale Ende des Verriegelungselements (15)(76) eine Verriegelungseinrichtung aufweist, die von einem überstehenden Anschlag (24) gebildet wird, der geformt ist, um unter dem Sicherungsanschlag (31) auf dem Element oder Instrument (26) in Eingriff zu treten, wenn das Verriegelungselement (15)(76) über dem Verbindungsteil (10) montiert wird, wobei das distale Ende (12) des Verbindungsteils (10) in der Bohrung (30) angeordnet wird und das Verriegelungselement (76)(15) gedreht wird, sowie Befestigungseinrichtungen (19,13)(71,81) zum lösbaren Befestigen des hülsenförmigen Verriegelungselements (15) (76) am Verbindungsteil (10).

2. Vorrichtung nach Anspruch 1, **dadurch gekennzeichnet, dass** sie eine elastisch vorgespannte Einrichtung einschließt, um den überstehenden Anschlag (24) unter dem Sicherungsanschlag (31) in Position zu halten.

3. Vorrichtung nach einem der vorangehenden Ansprüche 1 oder 2, **dadurch gekennzeichnet, dass** die Befestigungseinrichtung (19)(13) zum lösbaren Befestigen des Verriegelungselements am Verbindungsteil (10) einen radial verlaufenden geformten Schlitz (19) umfasst, mit dem ein Halteteil (13) auf dem Verbindungsteil (10) in Eingriff treten kann.

4. Vorrichtung nach einem der vorangehenden Ansprüche 1 oder 2, **dadurch gekennzeichnet, dass** die Befestigungseinrichtung (71)(81) zum lösbaren Befestigen des Verriegelungselements (76) am Verbindungsteil (10) einen radial verlaufenden geformten Schlitz (71) umfasst, der auf dem Verbindungsteil (10) vorgesehen ist und mit dem ein Halteteil (81) auf dem Verriegelungselement (36) in Eingriff treten kann.

5. Vorrichtung nach Anspruch 3 oder Anspruch 4, **dadurch gekennzeichnet, dass** der radial verlaufende geformte Schlitz (19) im Verriegelungselement (10) oder der Schlitz (71) auf dem Verbindungsteil (10) eine in Umfangsrichtung verlaufende Komponente (73) aufweist, in die das Halteteil (13) gedreht werden kann, um das Verriegelungselement (101)(76) auf dem Verbindungsteil (10) zu befestigen und zu verriegeln.

6. Vorrichtung nach Anspruch 5, **dadurch gekennzeichnet, dass** es eine elastisch vorgespannte Einrichtung (22) zum Festhalten des Halteteils (13) in der gedrehten Stellung im Schlitz (19)(71) einschließt.

7. Vorrichtung nach einem der vorangehenden Ansprüche, **dadurch gekennzeichnet, dass** die Bedienungseinrichtung einen Griff (90), Amboss (91) oder Einschlaghammer (5) umfasst.

8. Vorrichtung nach einem der vorangehenden Ansprüche, **dadurch gekennzeichnet, dass** das chirurgische Instrument oder Werkzeug einen proximalen Impaktor, einen Probeschaft oder eine Knochenraspel oder -feile umfasst.

## Revendications

1. Dispositif pour fixer de façon libérable des moyens d'actionnement (5) à un élément ou outil chirurgical (26), dont l'extrémité proximale est formée avec un perçage (30) et une butée de fixation (31), ledit dispositif comprenant un élément de raccordement (10) dont l'extrémité proximale est fixée aux moyens d'actionnement (5) ou comporte des moyens de fixation (11) pour fixer celle-ci aux moyens d'actionnement (5), et une extrémité distale (12) qui est adaptée pour rentrer à l'intérieur du perçage (30) dans l'élément ou outil chirurgical (26), **caractérisé par** :
l'inclusion d'un élément de verrouillage en forme de manchon (15), (76) qui peut coulisser et tourner sur l'élément de raccordement (10),
le fait que l'extrémité distale dudit élément de verrouillage (15), (76) comporte des moyens de verrouillage formés par une butée saillante (24) qui est conformée de façon à venir en prise en dessous de ladite butée de fixation (31) sur l'élément ou outil (26) lorsque l'élément de verrouillage (15), (76) est assemblé sur l'élément de raccordement (10), que l'extrémité distale (12) de l'élément de raccordement (10) est disposée dans le perçage (30), et que l'on fait tourner l'élément de verrouillage (76), (15), et que des moyens de fixation (19, 13), (71, 81) sont présents pour fixer de façon libérable ledit élément de verrouillage en forme de manchon (15), (76) audit élément de raccordement (10).

2. Dispositif selon la revendication 1, **caractérisé en ce qu'**il inclut des moyens sollicités élastiquement pour maintenir ladite butée saillante (24) en position en dessous de ladite butée de fixation (31).

3. Dispositif selon l'une quelconque des revendications 1 ou 2 qui précèdent, **caractérisé en ce que** les moyens de fixation (19), (13) pour fixer de façon libérable ledit élément de verrouillage audit élément de raccordement (10) comprennent une fente ayant une forme s'étendant radialement (19) qui peut venir en prise avec un élément de maintien (13) sur ledit élément de raccordement (10).

4. Dispositif selon l'une quelconque des revendications 1 ou 2 qui précèdent, **caractérisé en ce que** les moyens de fixation (71), (81) pour fixer de façon libérable ledit élément de verrouillage (76) audit élément de raccordement (10) comprennent une fente ayant une forme s'étendant radialement (71) disposée sur ledit élément de raccordement (10) et qui peut venir en prise avec un élément de maintien (81) sur ledit élément de verrouillage (36).

5. Dispositif selon la revendication 3 ou la revendication 4, **caractérisé en ce que** ladite fente ayant une forme s'étendant radialement (19) dans ledit élément de verrouillage (10), ou la fente (71) sur ledit élément de raccordement (10), comporte un composant s'étendant de façon circonférentielle (73) dans lequel on peut faire tourner l'élément de maintien (13) pour fixer et verrouiller ledit élément de verrouillage (101), (76) sur ledit élément de raccordement (10).

6. Dispositif selon la revendication 5, **caractérisé en ce qu'**il comprend des moyens sollicités élastiquement (22) pour maintenir ledit élément de maintien (13) dans la position tournée dans ladite fente (19), (71).

7. Dispositif selon l'une quelconque des revendications précédentes, **caractérisé en ce que** les moyens d'actionnement comprennent une poignée (90), une enclume (91) ou un marteau d'impact (5).

8. Dispositif selon l'une quelconque des revendications précédentes, **caractérisé en ce que** l'instrument ou outil chirurgical comprend un dispositif d'impact proximal, une tige de traction ou une râpe ou lime à os.
